# EUROPÄISCHE PATENTSCHRIFT

(11) **EP 1 442 087 B1**
(45) Veröffentlichungstag und Bekanntmachung des Hinweises auf die Patenterteilung: **24.01.2007**
(21) Anmeldenummer: 02802310.9
(22) Anmeldetag: 01.11.2002
(51) Int. Cl.: C09D 11/00, B41M 3/14, C12Q 1/68

(54) **MARKIERUNGSLÖSUNG ZUR FÄLSCHUNGSSICHEREN KENNZEICHNUNG EINES WERTGEGENSTANDS, AUS DER MARKIERUNGSLÖSUNG HERGESTELLTE MARKIERUNG SOWIE VERFAHREN ZUR MARKIERUNG EINES WERTGEGENSTANDS**
MARKING SOLUTION FOR COUNTERFEIT-RESISTANT IDENTIFICATION OF A VALUABLE OBJECT, MARKING PRODUCED BY THE MARKING SOLUTION AND METHOD FOR MARKING A VALUABLE OBJECT
SOLUTION DE MARQUAGE POUR L'IDENTIFICATION INFALSIFIABLE D'UN OBJET DE VALEUR, MARQUAGE REALISE A L'AIDE DE CETTE SOLUTION DE MARQUAGE ET PROCEDE DE MARQUAGE D'UN OBJET DE VALEUR

(30) Priorität: 02.11.2001 DE 10153596
(43) Veröffentlichungstag der Anmeldung: 04.08.2004
(73) Patentinhaber: November Aktiengesellschaft Gesellschaft für Molekulare Medizin, 91056 Erlangen (DE); Bundesdruckerei GmbH, 10969 Berlin (DE)
(72) Erfinder: NIGGEMANN, Matthias, 10967 Berlin (DE); PAESCHKE, Manfred, 16352 Basdorf (DE); FRANZ-BURGHOLZ, Arnim, 14612 Falkensee (DE); SPICKERMANN, Wolfgang, 56070 Koblenz (DE); MUTH, Olivier, 12277 Berlin (DE); JOSTEN, André, 90429 Nürnberg (DE); BAUER, Georg, 90409 Nürnberg (DE); WALTER, Harald, 8903 Birmensdorf (DE); GRASSL, Björn, 90415 Nürnberg (DE); HASSMANN, Jörg, 91052 Erlangen (DE)
(74) Vertreter: Hiebsch, Gerhard F.
(86) Internationale Anmeldenummer: PCT/EP2002/012226
(87) Internationale Veröffentlichungsnummer: WO 2003/038000

(56) Entgegenhaltungen:
- WO-A-00/44891
- WO-A-94/16902
- WO-A-99/67358
- US-A- 5 139 812

## Beschreibung

Die Erfindung bezieht sich auf eine Markierungslösung zur fälschungssicheren Kennzeichnung eines Wertgegenstands, insbesondere eines Dokuments. Sie betrifft weiter eine aus der Markierungslösung hergestellte Markierung sowie ein Verfahren zur Markierung eines Wertgegenstands.

Bei einer Vielzahl von Wertgegenständen kann zur Sicherung der Authentizität der Gegenstände eine fälschungssichere Kennzeichnung von besonderer Bedeutung sein. Dies betrifft insbesondere Wert- und/oder Sicherheitsdokumente, wie beispielsweise Banknoten, Ausweispapiere oder dergleichen, aber auch Verpackungen für sensible Gegenstände, wie beispielsweise Arzneimittel.

Derartige Markierungen können auf der Basis von Nukleinsäuren vorgenommen werden, wobei eine Echtheitsüberprüfung der Markierung unter Verwendung von zur Markierungsnukleinsäuresequenz komplementären Nukleinsäuresequenzen in Kombination mit einer durch eine Reaktion der Markierungsnukleinsäuresequenz mit ihren Komplementären aktivierbaren Testsubstanz erfolgen kann.

Aus der DE 197 38 816 A1 ist es bekannt, zur Markierung an einen Feststoff gebundene Nukleinsäuren zu verwenden. Zur Identifizierung müssen die Nukleinsäuren allerdings vom Feststoff durch ein Extraktionsverfahren entfernt werden. Die in Lösung vorliegenden Nukleinsäuren müssen dann mittels einer spezifischen Reaktion, wie der Polymerase-Ketten-Reaktion (PCR), vervielfältigt werden. In nachfolgenden Schritten wird die vervielfältigte Nukleinsäuresequenz analysiert. Das Verfahren ist zeit- und arbeitsaufwendig und nicht für einen Nachweis der Echtheit vor Ort geeignet. Zudem ist eine Extraktion der zur Markierung aufgebrachten Nukleinsäuren nicht bei jedem Feststoff möglich oder erwünscht.

Ein weiteres Verfahren zum Identifizieren einer an einem Festkörper vorgesehenen Markierung ist aus der DE 198 11 730 A1 bekannt. Diese Markierung weist eine Nukleotidsequenz auf, bei der ein erstes Ende über Spacermoleküle kovalent an den Festkörper gebunden ist, und bei der an ein zweites Ende ein magnetisches Mittel gebunden ist. Die Herstellung modifizierter Oligonukleotide ist mit einem vergleichsweise hohen Aufwand verbunden. Die Nukleotidsequenz der Markierung wird mit einer zu ihr korrespondierenden Nukleotidsequenz eines Detektionsmittels, das an den Festkörper gebunden ist, in Kontakt gebracht. Die auf dem Festkörper befestigten Markierungsmoleküle, die nur eine begrenzt dicke Markierungsschicht für die Aufnahme von aktiven Markierungsmolekülen bilden, sind jedoch gegen eine mechanische Beanspruchung instabil und gegenüber Verschmutzung anfällig, was zu einem Stabilitätsverlust der Markierung führen kann. Ferner ist das aufwendige Verfahren nur für plane Oberflächen, die einen engen Kontakt von Markierung und Detektionsmittel ermöglichen, geeignet.

Aus der US 5 139 812 ist es bekannt, eine vorgegebene Nukleinsäure enthaltende Tinte zur fälschungssicheren Markierung von Gegenständen zu verwenden. Die Markierung ist an einer geheimen Stelle eines wertvollen Gegenstands aufgebracht. Um eine Mehrzahl von Gegenständen unterscheidbar zu markieren, werden mit der Tinte unterschiedliche Beschriftungen aufgebracht. Die Identifikation einer solchermaßen aufgebrachten Markierung erfolgt durch Bindung einer weiteren Nukleinsäure an die vorgegebene Nukleinsäure. Die gebundene Nukleinsäure kann mittels einer Farbreaktion oder aufgrund einer radioaktiven Markierung sichtbar gemacht werden. Bei diesem Konzept muss die Markierung mittels eines mehrstufigen und dementsprechend aufwendigen Verfahrens nachgewiesen werden. Diese Methode ist somit ebenfalls nicht im alltäglichen Gebrauch einsetzbar.

Die EP 0 745 690 A2 beschreibt sogenannte molecular beacons und deren Anwendung für die Hybridisierung. Eine Verwendung zur Detektion von Markierungen ist aus diesem Dokument nicht bekannt.

Die US 5 866 336 beschreibt mit einem Fluorophor markierte Primer. Die Primer werden mittels einer Polymerase-Ketten-Reaktion (PCR) amplifiziert. Im hybridisierten Zustand wird eine Rückfaltung der Primer ausgelöst. Das Fluoreszenzverhalten des am Primer vorgesehenen Fluorophors ändert sich damit. Das bekannte Verfahren ist für eine schnelle Identifikation einer Markierung ungeeignet, weil es die kosten- und zeitaufwendige Polymerase-Ketten-Reaktion (PCR) erfordert.

Allen diesen genannten Konzepten ist gemeinsam, dass sie durch die Verwendung von Nukleinsäuresequenzen als Markierungsträger grundsätzlich ein hohes Sicherheitspotential und insbesondere eine hohe Fälschungssicherheit gewährleisten können. Allerdings kommt bei ihnen, insbesondere im Hinblick auf den apparativen Aufwand bei der Echtheitsprüfung, ein Einsatz der Markierungsträger für die Sicherung alltagsrelevanter Gegenstände und/oder für die schnell, kostengünstig und unkompliziert durchführbare Identifizierung der Markierung vor Ort, beispielsweise bei staatlichen Stellen oder Apotheken, nicht in Betracht. Dies betrifft in besonderem Maße die fälschungssichere Kennzeichnung von Dokumenten, bei denen bereits aufgrund des üblicherweise als Material eingesetzten Papiers besondere Sorgfalt bei einer Echtheitsprüfung geboten ist.

Der Erfindung liegt daher die Aufgabe zugrunde, eine Markierungslösung auf der Basis von Nukleinsäuresequenzen als Markierungsträger anzugeben, die auf besonders einfache Weise verwendbar und damit sowohl für den Einsatz für alltägliche Kennzeichnungen, insbesondere für die Kennzeichnung von Dokumenten, als auch für eine vergleichsweise einfache Identifizierung besonders geeignet ist. Des Weiteren soll ein für einen Einsatz im alltäglichen Bereich besonders geeignetes Verfahren zur fälschungssicheren Markierung eines Gegenstands angegeben werden.

Bezüglich der Markierungslösung wird diese Aufgabe erfindungsgemäß gelöst, indem sie als Komponenten eine wässrige Lösung mit einzelsträngigen Nukleinsäuren oder deren Derivaten sowie Glycerin und Polyethylenglykol (PEG) umfasst.

Die Erfindung geht dabei von der Überlegung aus, dass die Markierungslösung selbst bei einem vorgesehenen Einsatz im alltäglichen Bereich ein besonders hohes Maß an Fälschungssicherheit gewährleisten sollte. Dazu sollte die Markierungslösung unter Beibehaltung von Nukleinsäuresequenzen als Markierungsträger für einen robusten, alltäglichen Einsatz ertüchtigt werden. Eine besondere Vereinfachung bei der Handhabung eines derartigen Markierungsträgers im alltäglichen Bereich ist erreichbar, indem die Markierungslösung für ein Aufbringen durch ein Hochdruckverfahren, insbesondere durch Stempeln, geeignet ist.

Um dies zu ermöglichen, sind die als Markierungsträger vorgesehenen Nukleinsäuren gezielt mit Zuschlagmaterialien kombiniert, die eine Verwendung der Markierungslösung in der Art einer Stempelfarbe ermöglichen. Dabei sind die eingesetzten einzelsträngigen Nukleinsäuren oder deren Derivate ohne aufwendige Modifikationen vergleichsweise einfach und kostengünstig hergestellt. Die als Zuschlagmaterialien vorgesehenen Komponenten, nämlich Glycerin und Polyethylenglykol (PEG), sind vergleichsweise leicht verfügbar und untereinander und auch mit Nukleinsäuren besonders verträglich. Das Glycerin wirkt dabei als hygroskopische Substanz und hält den wässrigen Anteil in der Markierungslösung selbst bei längerer Lagerung in Umgebungsatmosphäre konstant. Es kann zudem als Haftvermittler wirken, während das Polyethylenglykol (PEG) eine gleichmäßige Aufbringung und Haftung der Markierungslösung auf einem zu markierenden Gegenstand erlaubt, indem es für eine ausreichende Viskosität der Markierungslösung sorgen und detergentienartig wirken kann.

Ein besonders zuverlässiger Fälschungs- oder Kopierschutz ist erreichbar, indem die Markierungslösung zusätzlich zum eigentlichen Markierungsträger diesem ähnliche, jedoch nicht als Markierungsträger vorgesehene Substanzen enthält, die für Uneingeweihte vom Markierungsträger nicht unterscheidbar sind. Bei einem Fälschungsversuch ist somit für einen Fälscher nicht erkennbar, welcher Bestandteil der Markierung in besonderem Maße nachzubilden ist, so dass - bei Nachbildung sämtlicher vorhandener Bestandteile - ein erheblicher Aufwand zu bewältigen wäre. Daher umfassen die Nukleinsäuren oder deren Derivate vorzugsweise einen als Markierungsträger vorgesehenen ersten Anteil und einen anderen zweiten Anteil. Dabei kann der zweite Anteil in der Art eines Hintergrunds dazu dienen, den eigentlich als Markierungsträger vorgesehenen ersten Anteil zu kaschieren.

Zu diesem Zweck liegt der nicht als Markierungsträger vorgesehene zweite Anteil zweckmäßigerweise im Überschuss in der Markierungslösung vor. Das Verhältnis des ersten Anteils zum zweiten Anteil beträgt für eine besonders hohe Fälschungssicherheit vorzugsweise 1:9 oder höher.

Für einen zuverlässigen Nachweis des als Markierungsträger vorgesehenen ersten Anteils der Nukleinsäuren oder deren Derivate durch Fluoreszenzreaktion mit ihren als sogenannte molecular beacons ausgestalteten Komplementären infolge Anregung mittels einer Lichtquelle, sollte der Markierungsträger in der Markierungslösung in ausreichend hoher, geeignet gewählter Konzentration vorliegen. In besonders vorteilhafter Weise ist dazu eine Konzentration des ersten Anteils in der Markierungslösung von 50 ppm bis 2000 ppm, vorzugsweise zwischen 200 ppm und 300 ppm, gewählt. Zum Nachweis des ppm-Werts kommen zweckmäßigerweise beispielsweise die Infrarot- oder Massenspektrometrie in Betracht.

Als einzelsträngige Nukleinsäuren sind vorzugsweise Desoxyribonukleinsäuren (DNAs) eingesetzt.

Für eine besonders fälschungssichere und praktikable Handhabbarkeit, insbesondere bezüglich ihrer Konsistenz, umfasst die Markierungslösung vorzugsweise 30-50 % der wässrigen Lösung mit einzelsträngigen Nukleinsäuren oder deren Derivaten sowie 20-40 % Glycerin und 10-40 % Polyethylenglykol (PEG).

Als hygroskopische Komponente können zwar Lithiumchlorid in einer Konzentration von 0,1 bis 3,0 M, Natrium- oder Kaliumlactat (0,5-5 %), Propylenglykol (1-10 %), Hyaluronsäure (0,2-2 %), Magnesiumchlorid (2-20 %) oder Sorbitol (1-10 %) eingesetzt werden, die allerdings nicht so gut verträglich zu den Nukleinsäuren oder deren Derivaten sind und zudem nicht als Haftvermittler fungieren können.

Um ein ortsgenaues Aufbringen der Markierungslösung zu erleichtern oder auch um gegebenenfalls zusätzlich zur Sicherungsfunktion auch reine Informationszwecke zu erfüllen, ist zweckmäßigerweise eine farbgebende Komponente eingesetzt. Wegen ihrer Licht- und Hitzebeständigkeit sowie ihrer chemischen Stabilität sind der Markierungslösung dafür vorzugsweise Pigmente in einer Konzentration von vorteilhafterweise bis zu 20 % zugesetzt.

Bezüglich der Markierung wird die genannte Aufgabe gelöst, indem die Markierungslösung eine Markierungsschicht bildet in einer Schichtdicke von 0,5 µm bis 5 µm. Die durch die Verwendung der Markierungslösung erhältliche langzeitstabile, haltbare und abriebfeste Markierungsschicht ermöglicht noch nach Jahren einen quantitativen Nachweis des Markierungsträgers und eine schnelle Identifizierung der Markierung vor Ort, d. h. zum Beispiel bei staatlichen Stellen, mit vergleichsweise einfachen, unkomplizierten und unempfindlichen Geräten, beispielsweise geeigneten Handlesegeräten.

In einer weiteren Ausgestaltung ist die Markierungsschicht vorzugsweise in einer vorgegebenen geometrischen Anordnung auf einem zu markierenden Gegenstand aufgebracht. Bei der geometrischen Anordnung kann es sich um ein vorgegebenes Muster, z. B. einen Barcode, handeln. Ein solches Muster lässt sich bei geeigneter Größe mehrmals auf Echtheit prüfen.

Zum Schutz vor Kontaminationen ist die Markierungsschicht in besonders vorteilhafter Ausgestaltung zumindest abschnittsweise mit einer Schutzschicht überdeckt. Um beispielsweise eine fluoreszenzoptische Identifizierung der Markierungsschicht zu ermöglichen, ist die Schutzschicht vorzugsweise transparent ausgebildet.

Bezüglich des Verfahrens zur Markierung von Gegenständen wird die genannte Aufgabe gelöst, indem die Markierungslösung auf einem zu markierenden Gegenstand aufgebracht wird.

Für eine besonders einfach handhabbare und somit von jedermann durchführbare Markierung sowie zudem für einen reproduzierbaren Auftrag der Markierungslösung wird diese vorzugsweise mittels eines Hochdruckverfahrens aufgebracht, wobei in besonders vorteilhafter Weiterbildung ein Stempel eingesetzt wird. Gerade die Wahl eines Stempels gewährleistet dabei eine konstante, gleichmäßige und bestimmte Konzentration des Markierungsträgers bei der Aufbringung der Markierungslösung auf den zu markierenden Gegenstand und ermöglicht zudem einen unkomplizierten Einsatz im alltäglichen Bereich.

Zur Verbesserung des optischen Eindrucks kann ein Stempel mit zweigeteiltem Stempelkissen verwendet werden. Der den Markierungsträger enthaltende Teil kann aus Kostengründen kleiner sein als ein bildgebender Teil, dem z. B. eine Rotfärbung hervorrufende Pigmente beigefügt sind, und einen für das Auge rot erscheinenden Abdruck erzeugt. Der bildgebende Teil ist vorzugsweise in den Farben grün, blau oder schwarz ausgebildet. Die Markierungslösung kann auch als klare Lösung aufgebracht werden, was einen unsichtbaren und somit geheimen Stempelabdruck bewirkt. Die Markierung kann ebenfalls über maschinelle, meist tintenstrahlbasierte, Stempeleinrichtungen aufgebracht werden.

In besonders vorteilhafter Ausgestaltung ist die Markierungslösung Teil eines Markierungskits. Dieser umfasst vorzugsweise einen mit der Markierungslösung befüllten Vorratsbehälter und ein Aufbringungsmittel, das eine an die Markierungslösung angepasste ldentifizierungslösung enthält.

Damit die ldentifizierungslösung einfach und schnell auf der Markierungsschicht aufgetragen werden kann, ist als Aufbringungsmittel vorteilhafterweise ein Stift vorgesehen. Die Spitze des Stifts ist dabei vorzugsweise aus einem saugfähigen Fasermaterial gebildet.

Zur schnellen und einfachen Identifizierung der Markierung vor Ort eignet sich die Detektion einer Fluoreszenzreaktion, die beim Inkontaktbringen der Markierungslösung und der Identifizierungslösung und beim Bestrahlen mit Licht einer vorgegebenen Wellenlänge ausgelöst wird. Zur Ermöglichung einer solchen Fluoreszenzreaktion umfasst die Identifizierungslösung vorzugsweise als sogenannte molecular beacons ausgestaltete Nukleinsäuren oder deren Derivate, die zu den als Markierungsträger vorgesehenen einzelsträngigen Nukleinsäuren oder deren Derivaten zumindest abschnittsweise komplementär ausgebildet sind.

Bei den molecular beacons sind die 3'- und 5'-Enden der Nukleinsäuren oder deren Derivate mit einem Fluorophor bzw. mit einem Fluoreszenzquenchermolekül modifiziert. Die terminalen Nukleotidsequenzen sind so gewählt, dass sie über einen kurzen Bereich komplementär sind und einen Doppelstrang ausbilden, während der Sequenzbereich in der Mitte des Oligonukleotids eine Biegung in der Art einer Haarnadel oder Schleife annimmt. Der Doppelstrang ist unter Normalbedingungen ausreichend stabil, um die terminalen Modifikationen in direkte Nachbarschaft zu bringen. Der damit geringe Abstand zwischen dem Fluorophor und dem Quencher ermöglicht einen strahlungslosen, die Fluoreszenz löschenden Strahlungsübergang nach dem sogenannten Förster-Effekt.

Ein derartiges Molekül kann zum Nachweis der Nukleinsäuresequenz des Markierungsträgers dienen. Dazu ist z. B. ein Sequenzbereich in dem Haarnadelabschnitt des molecular beacons komplementär zu dem Sequenzabschnitt des Markierungsträgers gewählt. Die Länge dieses Wechselwirkungsbereichs sollte ausreichend groß sein, damit genug Bindungsenergie freigesetzt werden kann, um den kurzen Doppelstrang im molecular beacon aufzubrechen. Unter diesen Voraussetzungen werden der Fluorophor und der Quencher räumlich getrennt, da sie sich jetzt an den entgegengesetzten Termini des neuen, stabileren Doppelstranges befinden. Als direkte Folge wird nun die Fluoreszenz nicht mehr gelöscht und kann nach entsprechender Anregung als Nachweis der Wechselwirkung mit der Nukleinsäuresequenz des Markierungsträgers dienen.

In weiterer vorteilhafter Ausgestaltung weist der Markierungskit einen mit der Markierungslösung befüllbaren Stempel auf. Dieser Stempel gewährleistet einen reproduzierbaren Auftrag der Markierungslösung auf den zu markierenden Gegenstand mit einer konstanten, gleichmäßigen und bestimmten Konzentration des Markierungsträgers.

Verwendung kann die Markierungslösung zweckmäßigerweise in allen Bereichen finden, in denen es um die Prüfung der Authentizität und um den Dokumentschutz geht. Daher ist die Markierungslösung vorzugsweise zur Markierung eines Wert- und/oder Sicherheitsdokuments, wie beispielsweise eines Ausweises, einer Banknote, oder einer Arzneimittelverpackung eingesetzt.

Die mit der Erfindung erzielten Vorteile bestehen insbesondere darin, dass die Markierungslösung auf der Basis von einzelsträngigen Nukleinsäuren oder deren Derivaten fälschungssicher und durch die Zuschlagmaterialien Glycerin und Polyethylenglykol (PEG) für einen gleichmäßigen und dauerhaften Auftrag mittels Stempels auf einem zu markierenden Gegenstand, insbesondere einem alltagsrelevanten Wert- oder Sicherheitsdokument, besonders geeignet ist. Gerade die durch den Stempelabdruck mit der Markierungslösung gebildete 0,5 µm bis 5 µm dicke Markierungsschicht mit einem 9-fachen Überschuss an Nukleinsäuren oder deren Derivaten, die nicht als Markierungsträger vorgesehen sind, gewährleistet einen besonders zuverlässigen Fälschungs- oder Kopierschutz, da darin die als Markierungsträger vorgesehenen Nukleinsäuren oder deren Derivate für Uneingeweihte unauffindbar sind. Des Weiteren ermöglicht diese langzeitstabile Markierungsschicht noch nach Jahren einen quantitativen Nachweis des Markierungsträgers und eine schnelle fluoreszenzoptische Identifizierung der Markierung vor Ort, d. h. zum Beispiel bei staatlichen Stellen, mit vergleichsweise einfach bedienbaren Geräten.

Ein Ausführungsbeispiel der Erfindung wird anhand einer Zeichnung näher erläutert. Darin zeigen:
- Fig. 1: eine Draufsicht auf eine fälschungssichere Markierung,
- Fig. 2: eine Seitenansicht auf einen Stempel,
- Fig. 3: die Spezifität des Signals in Abhängigkeit von der DNA-Sequenz,
- Fig. 4: das Fluoreszenzsigrtal der Markierung nach Aufbringen der Identifizierungslösung aufgenommen mit einem geeigneten Handlesegerät,
- Fig. 5a-5c: den Verfahrensablauf in schematischen Querschnittsansichten und
- Fig. 6: schematisch ein Beispiel für das mehrmalige Auslesen der Markierung.

Gleiche Teile sind in allen Figuren mit denselben Bezugszeichen versehen.

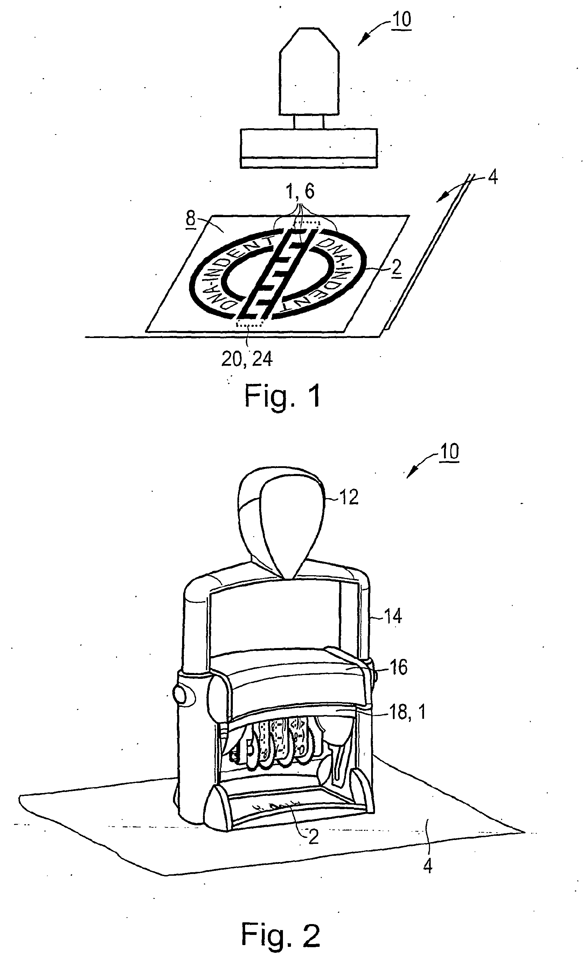

In Fig. 1 ist ein mit einer Markierungslösung 1 gebildeter Stempelabdruck 2 gezeigt, weicher auf einem Gegenstand 4, insbesondere einem Wertdokument, z. B. einer Banknote, aufgebracht ist. Der Stempelabdruck 2 der Markierungslösung 1 bildet eine Markierungsschicht 6 auf der Oberfläche des Gegenstands 4. Mit 8 ist eine die Markierungsschicht 6 überdeckende, zum Schutz vor Kontaminationen eingesetzte transparente Schutzschicht gekennzeichnet.

Die vorgesehene Markierungslösung 1 gewährleistet ein besonders hohes Maß an Fälschungssicherheit. Dazu sind als Grundlage der Markierungslösung 1 für den Markierungsträger Nukleinsäuresequenzen eingesetzt. Des Weiteren ist eine besondere Vereinfachung bei der Handhabung im alltäglichen Bereich erreicht, indem die Markierungslösung 1 für ein Aufbringen durch ein Hochdruckverfahren, insbesondere mittels eines Stempels 10, in der Art einer Stempelfarbe geeignet ist.

Als Stempel 10 ist im Ausführungsbeispiel ein herkömmlicher Handstempel eingesetzt, der, wie in Fig. 2 dargestellt, über einen an einem Handgriff 12 angeordneten Rahmen 14 ein zur Aufnahme der Markierungslösung 1 vorgesehenes feststehendes Stempelkissen 16 mit Stempelplatte 18 aufweist. Durch Drücken des Handgriffs 12 wird die durch das Stempelkissen 16 mit Markierungslösung 1 beaufschlagte Stempelplatte 18 um 180° gedreht und auf den zu markierenden Gegenstand 4 bewegt. Beim Auftreffen auf dem zu markierenden Gegenstand 4 wird der Stempelabdruck 2 der Markierungslösung 1 gebildet. Selbstverständlich sind auch andere handelsübliche Stempel einsetzbar.

Die Markierungslösung 1 zur fälschungssicheren Markierung ist im Ausführungsbeispiel besonders für ein Aufbringen durch Stempeln ertüchtigt. Daher sind der Markierungslösung 1 auf der Basis von 40 % einer wässrigen Lösung mit einzelsträngigen Nukleinsäuren oder deren Derivaten, insbesondere Desoxyribonukleinsäuren (DNA), Zuschlagstoffe zugegeben, die eine Verdunstung des wässrigen Anteils bei längerer Lagerung in Umgebungsatmosphäre durch hygroskopische Eigenschaften kompensieren und für zuverlässige und gleichmäßige Haftungseigenschaften sorgen. Zu diesem Zweck enthält die Markierungslösung 1 im Ausführungsbeispiel 20-30 % Glycerin und 10-30 % Polyethylenglykol (PEG).

Der Markierungslösung 1 sind weiterhin Pigmente in einer Konzentration von bis zu 10 % zugesetzt, die wegen ihrer Farbgebung das Aufbringen der Markierungslösung 1 auf dem zu markierenden Gegenstand 4 erleichtern und Informationszwecken dienen.

Die im Ausführungsbeispiel eingesetzten Desoxyribonukleinsäuren (DNAs) umfassen einen als Markierungsträger vorgesehenen ersten Anteil und einen anderen, diesem ähnlichen, jedoch nicht als Markierungsträger vorgesehenen zweiten Anteil. Der im Ausführungsbeispiel im 9-fachen Überschuss zugesetzte zweite Anteil sorgt in der Art eines Hintergrunds für eine erhöhte Fälschungssicherheit, indem sich der als Markierungsträger vorgesehene erste Anteil, im Ausführungsbeispiel mit einer Konzentration von 200 ppm bis 300 ppm in der Markierungslösung 1, in dem zweiten Anteil "verstecken" kann und somit für potentielle Fälscher schlecht auffindbar und somit nur schwer nachbildbar ist.

Die auf dem zu markierenden Gegenstand 4 im Ausführungsbeispiel mittels des Stempels 10 aufgebrachte Markierungslösung 1 bildet die Markierungsschicht 6 in einer Schichtdicke von 1 µm bis 2 µm. Diese langzeitstabile, haltbare und abriebfeste Markierungsschicht 6 ermöglicht noch nach Jahren einen quantitativen Nachweis des Markierungsträgers und eine schnelle Identifizierung der Markierung vor Ort.

Zur Identifizierung der Markierung wird eine ldentifizierungslösung 20 mit einem in Fig. 1 nicht näher dargestellten Aufbringungsmittel 22, das beispielsweise als Stempel, Dispenser, Stift oder ein anderes geeignetes Mittel ausgebildet sein kann, auf die Markierungsschicht 6 im Bereich einer gestrichelt angedeuteten Auftragfläche 24 aufgebracht. Die ldentifizierungslösung 20 umfasst als molecular beacons ausgestaltete Nukleinsäuren oder deren Derivate, die zu den als Markierungsträger vorgesehenen DNAs der Markierungslösung 1 zumindest abschnittsweise komplementär ausgebildet sind.

Die molecular beacons können mit einem NIR-Fluorophor und einem dafür geeigneten Quencher am 3' bzw. 5' Ende versehen sein. Zweckmäßigerweise wird als Fluorophor Cy 5 (Amersham) und als Quencher BHQ 3 (Biosearch Technologies Inc.) verwendet. Bei der Hybridisierung mit dem Markierungsträger kommt es zu der vorstehend näher beschriebenen Änderung der Sekundärstruktur des molecular beacons. Eine am molecular beacon vorgesehene fluorophore Markierung kann mittels einer Anregungslichtquelle 26, z. B. eine Laserdiode, angeregt werden. Das vom Fluorophor abgestrahlte Fluoreszenzlicht kann mittels einer Photodiode detektiert werden. Das Auftreten des charakteristischen Fluoreszenzsignals zeigt die Authentizität der Markierung an.

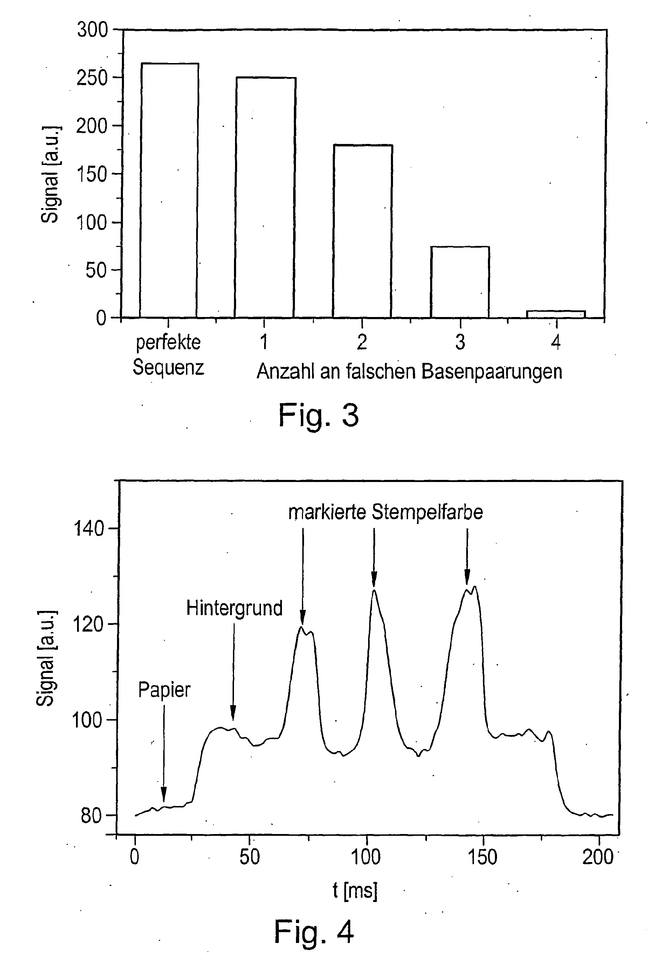

Fig. 3 zeigt im Vergleich die Stärke eines Fluoreszenzsignals, welche auf der Y-Achse angegeben ist. Das Fluoreszenzsignal entsteht bei der Hybridisierung der als Markierungsträger vorgesehenen DNA mit den zumindest abschnittsweise komplementär und als molecular beacons mit Fluorophoren ausgestalteten Nukleinsäuren oder deren Derivaten der ldentifizierungslösung 20. Dargestellt sind die Signale abzüglich des Hintergrunds einer Hybridisierung mit einem molecular beacon, wobei entweder keine oder ein, zwei, drei oder vier Basenfehlpaarungen entlang des hybridisierten Abschnitts auftreten. Bereits eine Fehlpaarung von zwei Basen ist mit dem vorliegenden Verfahren unterscheidbar. Eine Fehlpaarung von vier Basen führt zu einem drastisch niedrigeren Signal. Das belegt die hohe Spezifität des Identifikationsverfahrens.

In Fig. 4 ist das Signal eines mit DNA markierten Stempelabdrucks 2 nach Aufbringen der Identifizierungslösung 20 dargestellt. Die Identifizierungslösung 20 deckt hierbei drei aufeinanderfolgende Stege des ersten Stempelabdrucks 2 ab. Ausgelesen wurde hier mit einem geeigneten Handlesegerät, welches wie ein handelsüblicher Scanner über den Stempelabdruck 2 bewegt wurde. Im Überlappungsbereich des Stempelabdrucks 2 mit der Identifizierungslösung 20 treten Signalmaxima auf; in den Zwischenbereichen werden lediglich die Hintergrundfluoreszenzen als lokale Minima detektiert. Die Quotienten aus den jeweiligen lokalen Maxima geteilt durch den Wert der Hintergrundfluoreszenz zeigen hierbei nur eine geringe Schwankung von 10 % gegenüber einem Mittelwert. Somit ist gewährleistet, dass die Markierung sequenzspezifisch ausgelesen werden kann.

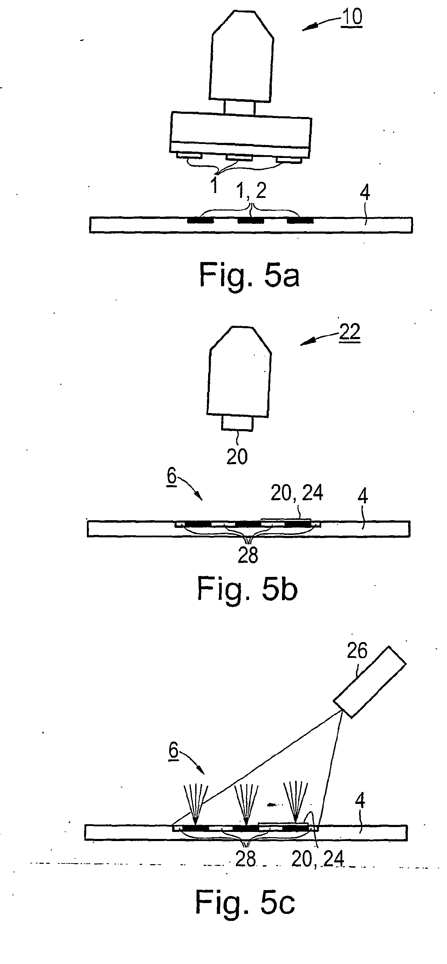

Die Fig. 5a bis 5c zeigen schematische Querschnittsansichten eines Ausführungsbeispiels des Verfahrens zur Markierung von Gegenständen 4, zur Identifizierung der Markierung und zur Detektion. Mittels des Stempels 10 wird auf dem Gegenstand 4, insbesondere einem Sicherheitsdokument, ein Stempelabdruck 2 aufgebracht. Die Identifizierungslösung 20 ist mit dem Aufbringungsmittel 22, im Ausführungsbeispiel mit einem Stift, in einer Menge von 1 µl aufgenommen. Die Identifizierungslösung 20 enthält zweckmäßigerweise in einer Konzentration von 1 pmol/µl ein molecular beacon in einem Dig Easyhyb-Puffer (Roche, Biomedicals). Der Lösung 20 kann ein gelber Farbstoff, z. B. der Lebensmittelfarbstoff E 104, zugesetzt sein. Die Identifizierungslö-sung 20 kann mit dem Aufbringungsmittel 22 auf die Auftragfläche 24 der Markierungsschicht 6 aufgebracht werden. Die Markierungsschicht 6 wird unterbrochen von Referenzflächen 28. Eine am molecular beacon vorgesehene fluorophore Markierung kann mittels der Anregungslichtquelle 26, z. B. eine Laserdiode, angeregt werden. Das Anregungslicht kann mit einem herkömmlichen polymerischen Filter Roscolene 862 - True Blue - (Rosco) gefiltert werden. Das vom Fluorophor abgestrahlte Fluoreszenzlicht infolge der Änderung der Sekundärstruktur des molecular beacons nach der Hybridisierung mit dem Markierungsträger kann mittels einer Photodiode detektiert werden. Das Auftreten des charakteristischen Fluoreszenzsignals zeigt die Authentizität der Markierung an.

Wie aus den Fig. 5a bis 5c ersichtlich ist, kann die Authentizität der Markierung schnell und einfach vor Ort geprüft werden. Der Prüfvorgang nimmt lediglich etwa 10 Sekunden in Anspruch. Es ist kein Waschvorgang oder kein Entfernen der Markierung vom markierten Gegenstand 4 erforderlich. Das vorgeschlagene Verfahren sowie die fälschungssichere Markierung eignet sich hervorragend zur Markierung von Massenprodukten, Sicherheitsdokumenten und dergleichen. Deren Identifizierung kann mit einem kostengünstig produzierten Handgerät erfolgen.

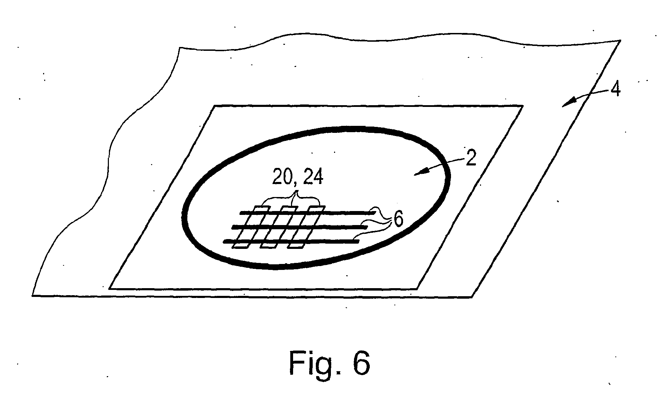

In Fig. 6 ist schematisch in Draufsicht ein Beispiel für ein mehrmaliges Identifizieren einer Markierung gezeigt. Ein Stempelabdruck 2 weist neben anderen Gestaltungsmerkmalen drei parallele Linien als Markierungsschicht 6 auf. Die Prüfung auf Authentizität des Stempelabdrucks 2 erfolgt mittels der Identifizierungslösung 20. Die Identifizierungslösung 20 wird im Ausführungsbeispiel orthogonal zu den drei parallelen Linien mittels des (nicht in Fig. 6 dargestellten) Aufbringungsmittels 22 auf die Auftragflächen 24 aufgebracht. Hierbei wird nur ein geringer Teil der Markierungsschicht 6 mit der Identifizierungslösung 20 versetzt. Für weitere Überprüfungen der Authentizität der Markierung können die noch nicht mit ldentifizierungslösung 20 versehenden Teilbereiche der Markierungsschicht 6 genutzt werden. Dieser Vorgang ist in Fig. 6 für ein dreimaliges Auslesen der Markierung dargestellt. Vorzugsweise ist die Identifizierungslösung 20 mit einem Farbstoff versetzt, um bereits überprüfte Teilbereiche der Markierung zu erkennen.

### Bezugszeichenliste

- 1: Markierungslösung
- 2: Stempelabdruck
- 4: Gegenstand
- 6: Markierungsschicht
- 8: Schutzschicht
- 10: Stempel
- 12: Handgriff
- 14: Rahmen
- 16: Stempelkissen
- 18: Stempelplatte
- 20: ldentifizierungslösung
- 22: Aufbringungsmittel
- 24: Auftragfläche
- 26: Anregungslichtquelle
- 28: Referenzfläche

## Patentansprüche

1. Markierungslösung (1), die als Komponenten eine wässrige Lösung mit einzelsträngigen Nukleinsäuren oder deren Derivaten sowie Glycerin und Polyethylenglykol (PEG) umfasst.

2. Markierungslösung (1) nach Anspruch 1, bei der die einzelsträngigen Nukleinsäuren oder deren Derivate einen als Markierungsträger vorgesehenen ersten Anteil und einen anderen zweiten Anteil Nukleinsäuren umfassen.

3. Markierungslösung (1) nach Anspruch 2, bei der das Verhältnis des ersten Anteils zum zweiten Anteil größer gleich 1:9 beträgt.

4. Markierungslösung (1) nach Anspruch 2 oder 3, bei der die Konzentration des ersten Anteils 50 ppm bis 2000 ppm beträgt.

5. Markierungslösung (1) nach einem der Ansprüche 1 bis 4, bei der als einzelsträngige Nukleinsäuren Desoxyribonukleinsäuren (DNAs) eingesetzt sind.

6. Markierungslösung (1) nach einem der vorhergehenden Ansprüche, die 30-50 % der wässrigen Lösung mit einzelsträngigen Nukleinsäuren oder deren Derivaten sowie 20-40 % Glycerin und 10-40 % Polyethylenglykol (PEG) umfasst.

7. Markierungslösung (1) nach einem der vorhergehenden Ansprüche, der Pigmente in einer Konzentration von bis zu 20 % zugesetzt sind.

8. Markierung mit einer durch eine Markierungslösung (1) nach einem der vorhergehenden Ansprüche gebildeten Markierungsschicht (6) in einer Schichtdicke von 0,5 bis 5 µm.

9. Markierung nach Anspruch 8, bei der die Markierungsschicht (6) in einer vorgegebenen geometrischen Anordnung auf einem zu markierenden Gegenstand (4) aufgebracht ist.

10. Markierung nach Anspruch 8 oder 9, deren Markierungsschicht (6) zumindest abschnittsweise mit einer Schutzschicht (8) überdeckt ist.

11. Markierung nach Anspruch 10, bei der die Schutzschicht (8) transparent ausgebildet ist.

12. Verfahren zur Markierung von Gegenständen (4), bei dem eine Markierungslösung (1) nach einem der Ansprüche 1 bis 7 oder eine Markierung nach einem der Ansprüche 8 bis 11 auf einem zu markierenden Gegenstand (4) aufgebracht wird.

13. Verfahren zur Markierung von Gegenständen (4) nach Anspruch 12, bei dem die Markierungslösung (1) auf dem zu markierenden Gegenstand (4) mittels eines Hochdruckverfahrens aufgebracht wird.

14. Verfahren zur Markierung von Gegenständen (4) nach Anspruch 12 oder 13, bei dem die Markierungslösung (1) mittels eines Stempels (10) aufgebracht wird.

15. Markierungskit mit einem mit einer Markierungslösung (1) nach einem der Ansprüche 1 bis 7 befüllten Vorratsbehälter und mit einem eine an die Markierungslösung (1) angepasste Identifizierungslösung (20) enthaltenen Aufbringungsmittel (22).

16. Markierungskit nach Anspruch 15, bei dem als Aufbringungsmittel (22) ein Stift vorgesehen ist.

17. Markierungskit nach Anspruch 15 oder 16, bei dem die Identifizierungslösung (20) als molecular beacons ausgestaltete Nukleinsäuren oder deren Derivate umfasst, die zu denen als Markierungsträger vorgesehenen zumindest abschnittsweise komplementär ausgebildet sind.

18. Markierungskit nach einem der Ansprüche 15 bis 17, mit einem mit der Markierungslösung (1) befüllbaren Stempel (10).

19. Verwendung einer Markierungslösung (1) nach einem der Ansprüche 1 bis 7 oder einer Markierung nach einem der Ansprüche 8 bis 11 zur Markierung eines Wert- und/oder Sicherheitsdokuments oder einer Arzneimittelverpackung.

## Claims

1. Marking solution (1) which comprises as components an aqueous solution with single-stranded nucleic acids or derivatives thereof and glycerine and polyethylene glycol (PEG).

2. Marking solution (1) according to claim 1, in which the single-stranded nucleic acids or derivatives thereof comprise a first portion provided as marking carrier and a further second portion of nucleic acids.

3. Marking solution (1) according to claim 2, in which the ratio of the first portion to the second portion is greater than or equal to 1:9.

4. Marking solution (1) according to claim 2 or 3, in which the concentration of the first portion is 50 ppm to 2,000 ppm.

5. Marking solution (1) according to one of claims 1 to 4, in which deoxyribonucleic acids (DNAs) are used as single-stranded nucleic acids.

6. Marking solution (1) according to one of the preceding claims, which comprises 30-50 % of the aqueous solution with single-stranded nucleic acids or derivatives thereof and 20-40% of glycerine and 10-40% of polyethylene glycol (PEG).

7. Marking solution (1) according to one of the preceding claims, to which pigments are added in a concentration of up to 20%.

8. Marking using a marking layer (6) formed by a marking solution (1) according to one of the preceding claims in a layer thickness of 0.5 to 5 µm.

9. Marking according to claim 8, in which the marking layer (6) is applied to an object (4) to be marked in a preset geometric arrangement.

10. Marking according to claim 8 or 9, the marking layer (6) of which is covered at least in sections by a protective layer (8).

11. Marking according to claim 10, in which the protective layer (8) is designed to be transparent.

12. Process for marking objects (4), in which a marking solution (1) according to one of claims 1 to 7 or a marking according to one of claims 8 to 11 is applied to an object (4) to be marked.

13. Process for marking objects (4) according to claim 12, in which the marking solution (1) is applied to the object (4) to be marked by means of a relief printing process.

14. Process for marking objects (4) according to claim 12 or 13, in which the marking solution (1) is applied by means of a stamp (10).

15. Marking kit having a stock container filled with a marking solution (1) according to one of claims 1 to 7 and having an application means (22) containing an identification solution (20) adapted to the marking solution (1).

16. Marking kit according to claim 15, in which a pen is provided as application means (22).

17. Marking kit according to claim 15 or 16, in which the identification solution (20) comprises nucleic acids or derivatives thereof designed as molecular beacons, which are designed to be complementary at least in sections to those provided as marking supports.

18. Marking kit according to one of claims 15 to 17, having a stamp (10) which can be filled with the marking solution (1).

19. Use of a marking solution (1) according to one of claims 1 to 7 or a marking according to one of claims 8 to 11 for marking a valuable and/or security document or medicament packaging.

## Revendications

1. Solution de marquage (1) comprenant, en tant que composants, une solution aqueuse avec des acides nucléiques simple brin ou leurs dérivés, ainsi que de la glycérine et du polyéthylène glycol (PEG).

2. Solution de marquage (1) selon la revendication 1, dans laquelle les acides nucléiques simple brin ou leurs dérivés comprennent une première partie, prévue comme support de marquage, et une autre deuxième partie d'acides nucléiques.

3. Solution de marquage (1) selon la revendication 2, dans laquelle le rapport entre la première partie et la deuxième partie est supérieur ou égal à 1:9.

4. Solution de marquage (1) selon la revendication 2 ou 3, dans laquelle la concentration de la première partie est dans la fourchette de 50 ppm à 2000 ppm.

5. Solution de marquage (1) selon l'une des revendications 1 à 4, dans laquelle sont utilisés comme acides nucléiques simple brin les acides desoxyribonucléiques (ADN).

6. Solution de marquage (1) selon l'une des revendications précédentes, comprenant de 30 à 50 % de la solution aqueuse avec des acides nucléiques simple brin ou de leurs dérivés, ainsi que de 20 à 40 % de glycérine, et de 10 à 40 % de polyéthylène glycol (PEG).

7. Solution de marquage (1) selon l'une des revendications précédentes, à laquelle sont ajoutés des pigments en une concentration allant jusqu'à 20 %.

8. Marquage avec une couche de marquage (6) formée par une solution de marquage (1) selon l'une des revendications précédentes, en une épaisseur de couche de 0,5 à 5 µm.

9. Marquage selon la revendication 8, dans lequel la couche de marquage (6) est appliquée, en un agencement géométrique prédéterminé, sur un objet (4) à marquer.

10. Marquage selon la revendication 8 ou 9, dont la couche de marquage (6) est recouverte, au moins par tronçons, par une couche protectrice (8).

11. Marquage selon la revendication 10, dans lequel la couche protectrice (8) est transparente.

12. Procédé de marquage d'objets (4) dans lequel une solution de marquage (1) selon l'une des revendications 1 à 7, ou un marquage selon l'une des revendications 8 à 11, est appliqué(e) sur un objet (4) à marquer.

13. Procédé de marquage d'objets (4) selon la revendication 12, dans lequel la solution de marquage (1) est appliquée sur l'objet (4) à marquer, à l'aide d'un procédé à haute pression.

14. Procédé de marquage d'objets (4) selon la revendication 12 ou 13, dans lequel la solution de marquage (1) est appliquée au moyen d'un poinçon (10).

15. Kit de marquage comprenant un réservoir de stockage, rempli d'une solution de marquage (1) selon l'une des revendications 1 à 7, et un moyen d'application (22) contenant une solution d'identification (20) adaptée à la solution de marquage (1).

16. Kit de marquage selon la revendication 15, dans lequel un stylet est prévu comme moyen d'application (22).

17. Kit de marquage selon la revendication 15 ou 16, dans lequel la solution de marquage (20) comprend des acides nucléiques ou leurs dérivés, arrangés en sondes moléculaires, formés de façon complémentaire, au moins par segments, par rapport à ceux prévus comme supports de marquage.

18. Kit de marquage selon l'une des revendications 15 à 17, avec un poinçon (10) susceptible d'être rempli de la solution de marquage (1).

19. Utilisation d'une solution de marquage (1) selon l'une des revendications 1 à 7, ou d'un marquage selon l'une des revendications 8 à 11, pour le marquage d'un document de valeur et/ou de sécurité, ou bien d'un emballage de produits médicamenteux.
